# EUROPÄISCHE PATENTSCHRIFT

(11) **EP 2 467 125 B1**
(45) Veröffentlichungstag und Bekanntmachung des Hinweises auf die Patenterteilung: **23.11.2016**
(21) Anmeldenummer: 10747181.5
(22) Anmeldetag: 12.08.2010
(51) Int. Cl.: A61K 8/86, A61K 8/92, A61Q 19/10

(54) **PRODUKTE MIT MESSTECHNISCH NACHGEWIESENER OLFAKTORISCHER WIRKUNG ZUR BEEINFLUSSUNG DER PSYCHO-PHYSIOLOGISCHEN AUSGANGSLAGE**
PRODUCTS WITH TECHNICALLY PROVEN OLFACTORY EFFECT FOR INFLUENCING A PSYCHO-PHYSIOLOGICAL INITIAL SITUATION
PRODUITS DOTÉS D'UN EFFET OLFACTIF APPROUVÉ SELON LA TECHNIQUE DE MESURE POUR L'INFLUENCE DE LA POSITION DE SORTIE PSYCHO-PHYSIOLOGIQUE

(30) Priorität: 20.08.2009 EP 09168323
(43) Veröffentlichungstag der Anmeldung: 27.06.2012
(73) Patentinhaber: Kneipp GmbH, 97084 Würzburg (DE)
(72) Erfinder: WOHLFART, Rainer, 97320 Sulzfeld am Main (DE); EBERT, Rebecca, 97852 Schöllbrunn (DE)
(74) Vertreter: Keller, Günter
(86) Internationale Anmeldenummer: PCT/EP2010/061723
(87) Internationale Veröffentlichungsnummer: WO 2011/020761

(56) Entgegenhaltungen:
- EP-A1- 0 815 850
- EP-A1- 2 286 790
- WO-A1-2004/006882
- WO-A1-2008/050084
- WO-A1-2009/090355
- CH-A5- 677 604
- DE-A1- 4 419 470
- DE-U1-202004 018 321
- US-A- 5 397 497
- US-A- 5 766 628
- US-A1- 2004 063 604
- US-B1- 6 268 333
- US-B1- 6 447 788

## Beschreibung

Die vorliegende Erfindung betrifft kosmetische Reinigungs- und Pflegezusammensetzungen vorzugsweise zum Baden. In dem Bereich der Körperpflege sind verschiedenste Formen von Badezusätzen bekannt. Zu den Badezusätzen zählen Badesalze, klassische Wirkbäder, fette Ölbäder sowie Schaumbäder und Emulsionsbäder. Bekannt sind auch mehrphasige Zubereitungen, die in Ruhe erkennbar zweiphasig sind, durch kräftiges Schütteln homogenisiert werden können und sich nach einiger Zeit wieder abtrennen. Derartige Ölbäder weisen einen relativ hohen Anteil an verschiedenen Ölbestandteilen auf, die vor allem der Pflege der Haut dienen sollen.

Die vorliegende Reinigungs- und Pflegezusammensetzung ist der Kategorie der klassischen Wirkbäder zuzuordnen, die anteilig (einen geringen Anteil) fette Öle, ätherische Öle und Tenside, sowie Solubilisatoren aufweisen und definitionsgemäß eine messtechnisch objektivierbare psycho-physiologische Wirkung haben

Bei bekannten Reinigungs- und Pflegezusammensetzungen wird häufig großer Wert auf die Reinigungsleistung der Zusammensetzung und/oder auf pflegende Aspekte des Badezusatzes gelegt.

Die EP 0 815 850 beschreibt eine Badeöl-Zusammensetzung mit ätherischen Ölen, die ohne Tenside auskommt. Dafür weisen die Badeölzusammensetzungen einen hohen Anteil (50 %) an Ethanol auf. Die US 5,766,628 beschreibt eine Bade- und Duschzusammensetzung mit Vesikel-formenden Eigenschaften, die zu wenigstens 20 Gew.-% aus einem Pflanzenöl oder einer wachsartigen apolaren Substanz besteht.

Die US 6,447,788 beschreibt kosmetische Körperpflegezubereitungen, die wenigstens 20 Gew.-% Honig enthalten. In der US 5,397,497 werden Badezusätze offenbart, die wenigstens 10 Gew.-% eines kosmetisch wirksamen Öls oder einer Mischung hiervon beinhalten. In dem deutschen Gebrauchsmuster 20 2004 018 321 werden Körperpflegepräparate offenbart, die wenigstens 40 % Honig, wenigstens 40 % Tenside und bis zu 5 Gew.-% ätherische Öle aufweisen. Gegenstand der US 4,921,874 sind medizinische Badeöle, die 68-95 Gew.-% eines oder mehrerer physiologisch annehmbarer Öle enthalten. Die US 6,268,333 beschreibt Duftstoffe mit einem beruhigenden Effekt, die in verschiedenen Produkten wie Parfüms, Shampoos, Luftverbesserungsmitteln, Deodorants und anderem eingesetzt werden können. In der WO 2008/050084 werden Duftzusammensetzungen beschrieben, denen bestimmte Einflüsse auf die Stimmungslage nachgesagt werden. In der US-Anmeldung 2004/0063604 werden Parfüm-Zusammensetzungen beschrieben die in verschiedenen Zusammensetzungen Duftmaterialien enthalten, die entspannende, nicht entspannende und gegebenenfalls neutrale Wirkungen entfalten. Gegenstand der DE 44 19 470 ist ein Verfahren zur Herstellung von Haut- und/oder Haarpflegemitteln aus Tensiden, Alkohol, Ölen, Fettalkohol und Wasser, denen auch ätherische Öle zugesetzt werden können.

Aufgabe der vorliegenden Erfindung ist es, eine Reinigungs- und Pflegezusammensetzung bereitzustellen, die gute Reinigungsleistung verbunden mit hoher Hautpflegequalität bereitstellt und darüber hinaus positiv die Stimmung der die Reinigungs- und Pflegezusammensetzungen verwendenden Person in gewünschter Weise beeinflusst. Erreicht wird dies durch die Zugabe eines oder mehrerer ätherischer Öle und/oder einzelner natürlicher, naturidentischer oder synthetischer Duftstoffe in verhältnismäßig hoher Konzentration. Bei der Bereitstellung des Badewassers wird die Reinigungs- und Pflegezusammensetzung aufgelöst. Ebenso werden bei der Aufbringung der Zusammensetzung beim Duschen die erfindungsgemäß eingesetzten ätherischen Öle freigesetzt. Diese können dabei von der die Reinigungs- und Pflegezusammensetzung verwendenden Person olfaktorisch über die Geruchsorgane aufgenommen werden.

Diese Aufnahme erfolgt unter folgenden Voraussetzungen:
1. Sinneseindrücke werden als erstes in einer Struktur im Gehirn verarbeitet, die sich Thalamus nennt und als "Tor zum Bewusstsein" bezeichnet wird. Nicht so Gerüche. Die Nervenimpulse, die in den olfaktorischen Sinneszellen ausgelöst werden, gelangen auf direktem Wege in das so genannte Limbische System. Dieses System bildet eine wichtige Grundlage unserer Gefühle. Gerüche können daher unmittelbar Gefühle auslösen, ohne dass diese Sinneseindrücke bewusst verarbeitet werden müssten. Die entstandenen Gefühle gehen in der Regel mit körperlichen Veränderungen, d. h. physiologischen Reaktionen, einher. Je nach Gefühl wird beispielsweise der Herzschlag schneller bzw. langsamer, oder die Hände fangen an zu schwitzen.
2. Stechende oder beißende Aromen wie die von Meerrettich, Alkohol oder Tabak erregen noch eine andere Art von Wahrnehmungssystem: die freien Nervenendigungen der Nervus Trigeminus. Dieses System hat Verbindungen zu einer Struktur, die Wachheit, physiologische Erregung und Aktivierung steuert: die im Hirnstamm befindliche Formatio reticularis. Die Funktionsweise dieses Systems machte man sich z. B. mit der Verwendung von Riechsalzen zur Erweckung Ohnmächtiger zunutze.
3. Wirkstoffe aus der Atemluft werden über die Nasenschleimhaut und die Bronchialschleimhaut aufgenommen, und gelangen so in den Blutkreislauf. Dadurch können sie Effekte auf innere Organe ausüben, und auch nach Durchquerung der Blut-Hirn-Schranke auf das Zentrale Nervensystem wirken.

Der Gegenstand der vorliegenden Erfindung ist in den Patentansprüchen definiert. Es handelt sich um eine kosmetische Reinigungs- und/oder Pflegezusammensetzung vorzugsweise ein Badezusatz mit einer messtechnisch nachgewiesenen olfaktorischen, die psycho-physiologische Ausgangslage beeinflussenden Wirkung, die 30-70 Gew.-% mindestens eines Tensids und/oder Solubilisators und 0,1-20 Gew.-% einer Mischung mehrerer verschiedener ätherischer Öle und/oder einzelner natürlicher, naturidentischer oder synthetischer Duftstoffe enthält. Die erfindungsgemäßen Zusammensetzungen enthalten, abgesehen von den ätherischen Pflanzenölen, nur 0,1-5 Gew.-% an Pflanzenölen die pflegenden Charakter aufweisen, und bevorzugt keinen Honig.

In bevorzugter Ausführungsform enthält die Zusammensetzung zwischen 35 und 55 Gew.-% eines Tensids oder einer Mischung verschiedener Tenside und/oder eines Solubilisators, sowie 1,5-10 Gew.-% eines ätherischen Öls oder einer Mischung mehrerer verschiedener ätherischer Öle und/oder einzelner natürlicher, naturidentischer oder synthetischer Duftstoffe. Unter dem Begriff "Solubilisator" werden solche Lösungsvermittler verstanden, die die Verteilung der ätherischen Öle im Wasser gewährleisten. Eine klare Abgrenzung zwischen Tensid und Solubilisator ist häufig nicht möglich. Erfindungsgemäß wird der mengenmäßige Anteil des Tensids den Anteil des Solubilisators überwiegen. Beispiele für Solubilisatoren sind PEG 40-Sorbitan Peroleate oder PEG 40-Hydrogenated Castor Oil.

Als weiteren Bestandteil enthält die Reinigungs- und Pflegezusammensetzung 0,1-5 Gew.-%, bevorzugt 1-2 Gew.-% eines weiteren Pflanzenöls, beispielsweise Sonnenblumenöl, Olivenöl oder Arganöl. Der Anteil an Öl überschreitet jedoch 5 Gew.-% der Gesamtzusammensetzung nicht.

Weiterhin kann die Zusammensetzung 1-20 Gew.-%, bevorzugt 2-15 Gew.-% Glycerin enthalten. Das Glycerin wirkt einerseits als Viskositätsregler der fertigen Zusammensetzung und hat andererseits eine pflegende Wirkung auf die Haut.

Zur Stabilisierung der in der Reinigungs- und Pflegezusammensetzung verwendeten fetten und ätherischen Öle kann die Zusammensetzung in bevorzugter Ausführungsform bis 0,1% auf die zu stabilisierende Ölphase an Mischtocopherolkonzentrat 70% (natürlich) enthalten.

In bevorzugter Ausführungsform enthält die Reinigungs- und Pflegezusammensetzung keine Konservierungsstoffe, sowie keine Paraffine, Silikone und/oder Mineralöle.

In bevorzugter Ausführungsform enthält die Reinigungs- und Pflegezusammensetzung weiterhin einen oder mehrere Pflanzenextrakte. Geeignete Pflanzenextrakte stammen vom Hopfen, Baldrian, Melisse, Lavendel und/oder Rosmarin. Die Pflanzenextrakte werden in bevorzugter Ausführungsform durch CO₂-Extraktion gewonnen.

Manche Bestandteile, die üblicherweise in Reinigungs- und Pflegezusammensetzungen eingearbeitet werden, wie beispielsweise Detergenzien, stammen von tierischen Produkten her. So werden aus tierischem Fett, Talg, Knochen oder Haut durch chemische Behandlung waschaktive Substanzen, wie Seifen, hergestellt. Die erfindungsgemäße Reinigungs- und Pflegezusammensetzung besteht jedoch nur aus Komponenten, die aus pflanzlichen Produkten hergestellt wurden. Dabei enthält die Reinigungs- und Pflegezusammensetzung in bevorzugter Ausführungsform keine Bestandteile, die von Tieren herstammen.

Die erfindungsgemäße Reinigungs- und Pflegezusammensetzung beinhaltet als eine Hauptkomponente waschaktiven Substanzen, Tenside oder Solubilisatoren in einer Menge von 30-70 Gew.-%, bevorzugt 35-55 Gew.-% und besonders bevorzugt 38-47 Gew.-% bezogen auf die fertige Zusammensetzung. Tenside sind amphiphile Stoffe, die organische, unpolare Substanzen im Wasser lösen können. Bedingt durch ihren spezifischen Molekülaufbau mit mindestens einem hydrophilen und einem hydrophoben Molekülteil sorgen sie für eine Herabsetzung der Oberflächenspannung des Wassers, die Benetzung der Haut, die Erleichterung der Schmutzentfernung und Schmutzablösung, ein leichtes Abspülen und die Schaumverstärkung und Schaumregulierung.

Der hydrophile Anteil eines Tensidmoleküls wird meist durch polare funktionelle Gruppen, die von entsprechenden Säureresten herstammen, gebildet, wie beispielsweise -COO⁻, -OSO₃²⁻, -SO₃⁻. Der hydrophobe Teil des Tensids wird in der Regel durch unpolare Kohlenwasserstoffreste gebildet. Nach Art und Ladung des hydrophilen Molekülteils werden Tenside unterschieden nach anionischen Tensiden, amphoteren Tensiden bzw. nichtionischen Tensiden und kationischen Tensiden.

Im Rahmen der vorliegenden Erfindung werden bevorzugt anionische Tenside eingesetzt, wie Acyl-Isothionate, Alkylarylsulphonate, Alkylsulphonate, Sulphosuccinate sowie Schwefelsäureester, wie Alkylethersulfate oder Arylsulfate.

Vorteilhaft einzusetzende amphotere Tenside sind beispielsweise Acyl-/Dialkylethylendiamin. Als nichtionische Tenside werden bevorzugt eingesetzt Alkohole, Alkanolamide, Ester, die durch Veresterung von Carbonsäuren mit Ethylenoxid, Glycerin, Sorbitan oder anderen Alkoholen entstehen, sowie Ether, insbesondere ethoxylierte/propoxylierte Alkohole, ethoxylierte/propoxylierte Ester, ethoxylierte/propoxylierte Glycerinester. In besonders bevorzugter Ausführungsform wird als Hauptkomponente der waschaktiven Substanzen erfindungsgemäß Polyoxyethylen-Sorbitanmonolaureat (Tween 20) eingesetzt.

Neben der waschaktiven Komponente enthält die erfindungsgemäße Reinigungs- und Pflegezusammensetzung einen maßgeblichen Anteil eines ätherischen Öls oder eines Gemisches mehrerer verschiedener ätherischer Öle und/oder einzelner natürlicher, naturidentischer oder synthetischer Duftstoffe. Die erfindungsgemäß eingesetzten ätherischen Öle sind sowohl natürliche Produkte als auch naturidentische Nachstellungen von Hauptkomponenten ätherischer Öle, die direkt aus Pflanzen gewonnen werden. Aufgrund der Herstellung der ätherischen Öle aus Pflanzen sind die erfindungsgemäß eingesetzten ätherischen Öle immer Gemische verschiedener chemischer Verbindungen, die hinsichtlich der einzelnen Komponenten und des Verhältnisses dieser Komponenten zueinander beeinflusst werden durch die Herkunft der Pflanzen und durch die genauen Bedingungen des Gewinnungsverfahrens.

Zum großen Teil sind einzelne Komponenten der ätherischen Öle bekannt. Ätherische Öle werden hauptsächlich aus Pflanzen bzw. bestimmten Pflanzenteilen gewonnen. In chemischer Hinsicht unterscheiden sich die einzelnen Komponenten wesentlich voneinander. Es handelt sich bei den Bestandteilen der ätherischen Öle häufig um Kohlenwasserstoffe, Alkohole, Aldehyde, Ketone, Ester, Phenole, Phenylester und auch Verbindungen mit anderen komplexen Strukturen. Die Molekulargewichte der einzelnen Verbindungen sind relativ niedrig. Meistens überschreitet das Molekulargewicht nicht einen Wert zwischen 200 bis 300 Dalton.

Ein Gemisch aus von Citrusarten und Citronengras (im Handel auch als sogenannte Indische Melisse bezeichnet) gewonnenem ätherischem Öl, in Kombination mit Terpenen und Terpenoiden und Litsea Cubeba Öl. wird erfindungsgemäss eingesetzt. Die Verwendung eines derartigen Öls hat einen positiven Einfluss und hebt die Stimmungslage nachweislich.

Ein anderes ätherisches Öl wird aus der Minze gewonnen. Auch dieses Öl hat einen belebenden, die Stimmung positiv beeinflussenden und aktivierenden Effekt. Einen eher beruhigenden Effekt hat dagegen Baldrianöl, das aus der Baldrianpflanze (Valeriana Officinalis) gewonnen wird.

Zitronenöl kann aus der Schale von Citrusfrüchten gewonnen werden. Zitronenöl kann bis zu 95 % Monoterpenkohlenwasserstoffe, hauptsächlich Limonen, aber auch andere Terpene, wie α-Terpinen und β-Pininen enthalten. Auch hier hängt das Verhältnis der einzelnen Komponenten zueinander von der Zitronenart und dem Ursprungsland ab.

Citronellaöl wird aus verschiedenen aromatischen Gräsern durch Wasserdampfdestillation gewonnen. Es gibt verschiedene Typen von Citronellaöl, in Abhängigkeit vom Ursprungsland und der Art der Herstellung. Das Ceylon-Citronellaöl wird durch Dampfdestillation von frischen Blättern und Stengeln des Grases *Cymbopogon nardus* erhalten. Das Java-Citronellaöl wird durch Wasserdampfdestillation von Stengeln und Blättern des Grases *Cymbopogon winterianus* vor allem in Südosten Asiens, in Indien und in Indonesien gewonnen. Citronellaöl enthält vor allem Citronellal, Geraniol, Citronellol, Granylacetat, Citronellylacetat und viele andere Komponenten in geringeren Anteilen.

Litsea Cubebaöl ist das durch Wasserdampfdestillation aus den Früchten gewonnene ätherische Öl des Litseabaums (Litsea Cubeba Persoon) . Die Pflanze hat zitronenartig duftende Blätter und Blüten und zählt zu den Lorbeergewächsen. Die kleinen Früchte des Baumes sehen aus wie Pfefferfrüchte, daher der volkstümliche Name Cubebenpfeffer. Das ätherische Öl enthält hauptsächlich Citral (70 - 80%), Limonen (10 - 15%), Linalool, Geraniol, Nerol und wirkt aktivierend erfrischend und konzentrationsfördernd.

Einzelriechstoffe die sowohl natürlichen, naturidentischen oder synthetischen Ursprungs sein können, können die Duftwirkung der ätherischen Öle je nach Ihren spezifischen Eigenschaften verstärken, aber auch abschwächen. Entscheidend für Art und Intensität des Einflusses auf die psycho-physiologische Ausgangslage ist daher stets der olfaktorische Effekt der sich aus dem qualitativen und quantitativen Zusammenspiel aller vorhandenen Duftstoffe ergibt.

Die wesentlichen Bestandteile der erfindungsgemäßen kosmetischen Reinigungs- und Pflegezusammensetzung sind in der nachfolgenden Tabelle 1 zusammengestellt, wobei der bevorzugte und der besonders bevorzugte Bereich in Gew.-% angegeben sind. Die Gewichtsprozente sind als (Gewicht/Gewicht) angegeben und beziehen sich auf die fertige Zusammensetzung. Die Zusammensetzung wird immer mit gereinigtem Wasser auf 100 Gew.-% ergänzt. Die Mischung der ätherischen Öle wird in die kosmetische Reinigungs- und/oder Pflegezusammensetzung in einer Menge von 0,1 - 20 Gew.-%, bevorzugt 0,5 - 15 Gew.-% und besonders bevorzugt in einer Menge von 1,0 - 10 Gew.-% eingearbeitet.

**Tabelle 1**

| **Bestandteil** | **bevorzugter Bereich** | **besonders bevorzugter Bereich** |
|---|---|---|
| ätherische Öle, Mischungen ätherischer Öle und/oder einzelner natürlicher, naturidentischer oder synthetischer Duftstoffe | 0,1 - 15 | 1 - 10 |
| waschaktives Tensid | 35 - 70 | 40 - 60 |
| Pflanzenöl | 0 - 5 | 0,1 - 2 |
| Viskositätsregler (Glycerin) | 0 - 15 | 0 - 15 |
| Mischtocopherolkonzentrat | 0 - 0,1 | 0,01 - 0,1 |
| Farbstoff | 0 - 1,2 | 0,1 - 1,1 |
| Pflanzenextrakt | 0 - 1 | 0,01 - 1 |
| Wasser, gereinigt | ad 100 Gew.-% | ad 100 Gew.-% |

Mischungen ätherischer Öle, die erfindungsgemäß bevorzugt eingesetzt werden, beinhalten die in Tabelle 2 dargestellten Bestandteile:

**Tabelle 2**

| **Inhaltsstoffe** | **Cas Nr.** | **bevorzugter Bereich** | **besonders bevorzugter Bereich** |
|---|---|---|---|
| d-Limonen | 5989-27-5 | 40 % bis 50 % | 45% |
| Litsea Cubeba Oil (vorzugsweise aus China/Vietnam/Zaire) | 0068855-99-2 | 20 % bis 30 % | 25% |
| Citral | 0005392-40-5 | 20 % bis 30 % | 23 % |
| Lemon Oil (vorzugsweise aus Italien (Sizilien), Spanien, Argentinien) | 0008008-56-8 | 1 % bis 5 % | 2% |
| Sweet Orange Oil (bevorzugt: Brasilien, Florida) | 0068647-72-3 | 1 % bis 5 % | 2% |
| Citronella Oil (Java, bevorzugt: China) | 0008000-29-1 | 1 % bis 5 % | 1 % |
| Inhaltsstoffe unter 1 %: | | | |
| Anis Alcohol | 105-13-5 | < 1 % | |
| Farnesol | 4602-84-0 | < 1 % | |
| Linalool | 78-70-6 | < 1 % | |
| Citronellol | 106-22-9 | < 1 % | |
| Geraniol | 106-24-1 | < 1 % | |

Bevorzugte Ausführungsformen von kosmetischen Reinigungs- und/oder Pflegezusammensetzungen werden in den nachfolgenden Beispielen wiedergegeben:

**Beispiel 1**

| Handelsname | INCI/CTFA | Einwaage in Masse% | Verwendungszweck | Hersteller/Lieferant |
|---|---|---|---|---|
| Polysorbat 20 (Tween 20) | Polysorbate 20 | 50 - 60 | Solubilisator/ waschaktive Substanz | Evonik GmbH Goldschmidt GmbH, Goldschmidtstraße 100,45127 Essen |
| Wasser, gereinigt | Aqua (Water) | 30 - 40 | Wasser | |
| Pö Freshness | Parfum (Fragance) | 5 - 10 | Olfaktorisch wirksamer Bestandteil | Firma Michael Schmitt Andemacher Str.8 50968 Köln |
| Sonnenblumenöl | Helianthus Anuus (Sunflower) Seed oil | 0,1 - 1 | Pflanzenöl | Gustav Heess GmbH, Am Gewerbering 4/6l, 83533 Edling |
| Farbstoff Chinolinqelb | Chinolingelb CI 47005, E 104 | 0,1 - 1 | Farbstoff | Pharmorgana GmbH, An der Guldenmühle 7, 65817 Eppstein |
| Farbstoff gelb | FD& C yellow No. 5 CI 19140, E 140 | 0,01 - 0,1 | Farbstoff | BASF Chem Trade GmbH, Industriestr. 20, 91593 Burgbernheim |
| Mischtocopherolkonzentrat 70% (natürlich) | Tocopherol | 0,01 - 0,1 | Stabilisator | Cognis Deutschland GmbH, Henkestraße 67, 40551 Düsseldorf |
| Summe | | 100 | | |

Dieser Badezusatz ruft bei der Anwendung eine messtechnisch nachgewiesene, erfrischende, konzentrationsfördernde Wirkung und einen interessanten Eindruck hervor.

### Zusammensetzung Parfümöl (Pö) Freshness

**Tabelle 3**

| Inhaltstoff | CAS-Nummer | Anteil in Gew% |
|---|---|---|
| Terpenes and Terpenoids, limonen fraction | 65996-98-7 | 10 - 50 |
| Linalyl acetat | 115-95-7 | 10 - 50 |
| Litsea Cubeba Fruit Oil | 68855-99-2 | 10 - 50 |
| Rosewood Oil Bresil | 8015-77-8 | 5 - 10 |
| Cornmint oil | 68917-18-0 | 5 - 10 |
| Linalool | 78 -70 6 | 5 - 10 |
| β-Pinen | 127-91-3 | 1 - 5 |
| α-Pinen | 80-56-8 | 0,1 - 1 |
| γ-terpinene | 99-85-4 | 0,1 - 1 |
| Ocimene | 13877-91-3 | 0,1 - 1 |
| Spearmint Oil | 8008-79-5 | 0,1 - 1 |
| Terpinyl acetat | 80-26-2 | 0,1 - 1 |

**Beispiel 2**

| Handelsname | INCI/ CTFA | Einwaage in % | Verwendungzweck | Hersteller/Lieferant |
|---|---|---|---|---|
| Polysorbat 20 (Tween 20) | Polysorbate 20 | 50-70 | Solubilisator/ waschaktive Substanz | Evonik GmbH Gddschmidt GmbH, Goldschmidtstraße 100, 45127 Essen |
| Wasser, gereinigt | Aqua (Water) | 15-30 | Wasser | |
| Citronellöl Java 100 % Stpfl. | Cymbopogon Nardus (Citronella) Oil | 5-10 | dfaktorisch wirksamer Bestandteil | Düllberg Konzentra Obenhauptstr. 3 D - 22335 Hamburg |
| Parfümöl Gerarium | Parfum (Fraganoe) | 2-5 | dfaktaisch wirksamer Bestandteil | Düllberg Konzentra Obenhauptstr. 3 D-22335 Hamburg |
| Parfümöl (II) | Parfum (Fragrance) | 2-5 | dfaktorisch wirksamer Bestandteil | Firma Michael Schmitt Andernacher Str.8 50968 Köln |
| Baldrianöl 25 % Stpfl. | Parfum (Fragrance), Valeriana Officinalis Oil | 2-5 | dfaktorisch wirksamer Bestandteil | Düllberg Konzentra Obenhauptstr. 3D-22335 Hamburg |
| Hopfenesxtrakt 4-6:1 (CO2) | Humulus Lupulus Hops Extract | 0,1-1 | Pflanzenextrakt | Falvex Natuextrakte, Nadstraße 7 D - 66780 Rehlingen |
| Farbstoff blau (FD & C Blue No. 1, CI 42090) | CI 42090 (Blue 1) | 0,01 -0,1 | Farbstoff | Pharmorgana GmbH, An der Guldenmühle 7, 65817 Eppstein |
| Mischtocopherolkonzentrat 70% (natürlich) | Tocopherol | 0,01-01 | Stabilisator | Cognis Deutschland GmbH, Henkestraße 67, 40551 Düsseldorf |
| | | 100,0 | | |

Dieser Badezusatz hat eine positive, entspannende Wirkung und einen beruhigenden Effekt. Er sollte vorzugsweise vor dem Schlafengehen angewendet werden.

### Zusammensetzung Parfümöl (II)

**Tabelle 4**

| Inhaltstoff | CAS-Nummer | Anteil in Gew% |
|---|---|---|
| Eucalyptus globulus Leaf Oil | 8000-48-4 | 10 - 50 |
| Clove bud oil | 8000-34-8 | 10 - 50 |
| Lavendula Hybrida Oil | 8022-15-9 | 5 - 10 |
| Turpentine Oil | 9005-90-7 | 5 - 10 |
| Camphor | 21368-68-3 | 5 - 10 |
| Alpha-pinene | 80-56-8 | 5 - 10 |
| Alpha-terpineol | 98-55-5 | 1 - 5 |
| Camphene | 79-92-5 | 1 - 5 |
| dl-Borneol | 507-70-0 | 1 - 5 |
| Linalyl acetat | 115-95-7 | 1 - 5 |
| p - Cymene | 99-87-6 | 1 - 5 |
| Shiu oil | 8022-91-1 | 1 - 5 |
| Thym blanc | | 1 - 5 |
| Thymol | 89-83-8 | 1 - 5 |
| β-Pinen | 127-91-3 | 0,1 - 1 |
| Bornyl acetat | 76-49-3 | 0,1 - 1 |
| Dipentene | 138-86-3 | 0,1 - 1 |
| Isobornyl acetat | 125-12-2 | 0,1 -1 |
| Linalool | 78-70-6 | 0,1 - 1 |
| Myrcene | 123-53-3 | 0,1 - 1 |
| Weitere Parfümbestandteile | | < 0,1 |

### Zusammensetzung Parfümöl "Geranium"

**Tabelle 5**

| | | |
|---|---|---|
| Citronellol | | 50% |
| Citronellylformiat | | 16% |
| Geraniol | | 12% |
| Linalool | | 5% |
| Weitere Parfümbestandteile | | Ad 100% |

Zusammensetzung Baldrianöl 25%Stpfl

**Tabelle 6**

| | | |
|---|---|---|
| Baldrianöl | | 25% |
| Andere ätherische Öle | | 11% |
| Naturidentische Riechstoffe | | 64% |

**Beispiel 3**

| Handelsname | INCI/CTFA | Einwaage in Masse % | Verwendungszweck | Hersteller/Lieferant |
|---|---|---|---|---|
| PöZitrone-Melisse | Parfum (Fragance) | 1-5 | dfaktorisch wirksamer Bestandteil | Firma Michael Schmitt Andernacher Str.850968 Köln |
| Sonnenblunenöl | Helianthus Anuus (Sunflower) Seed oil | 0,1-1 | Pflanzenöl | Gustav Heess GmbH, Am Gewerbering 4/6l, 83533 Eding |
| Glycerol 85% | Glycerin | 10-15 | Viskositätssenkender Hautpflegestoff | Fauth & Co GmbH, Innstraße 35 -37, 68199 Mannheim |
| Polysorbat 20 (Tween 20) | Polysorbate 20 | 40-50 | Solubilisator/ waschaktive Substanz | Evonik GmbH Goldschmidt GmbH, Goldschmidtstraße 100, 45127 Essen |
| Mischtocopherolkonzentrat 70% (natürlich) | Tocopherol | 0,01- 0,1 | Stabilisator | Cognis Deutschland GmbH Henkestraße 67, 40651 Düsseldorf |
| Farbstoff Chinolingelb | Chinolingelb Cl 47005, E 104 | 0,1-1 | Farbstoff | Pharmorgana GmbH, An der Guldenmühle 7,65817 Eppstein |
| Farbstoff gelb | FD&C yellow No. 5 Cl 19140, E 140 | 0,01- 0,1 | Farbstoff | BASF Chem Trade GmbH, Industriestr. 20, 91593 Burgbernheim |
| Wasser, gereinigt | Aqua (Water) | 30-40 | Wasser | |
| Summe | | 100,0000 | | |

Dieser Badezusatz hat eine entspannende und stresslösende Wirkung. Er sollte bevorzugt nach körperlicher, seelischer oder emotionaler Anspannung angewendet werden.

### Zusammensetzung Parfümöl Zitrone-Melisse

**Tabelle 7**

| Inhaltstoff | CAS-Nummer | Anteil in Gew% |
|---|---|---|
| Terpenes and Terpenoids, limonen fraction | 65996-98-7 | 10 - 50 |
| Citral | 5392-40-5 | 10 - 50 |
| Litsea Cubeba Fruit Oil | 90063-59-2 | 10 - 50 |
| Citronella Oil | 8000-29-1 | 1 - 10, bevorzugt 5 - 10 |
| Lemon Oil | 8008-56-8 | 1 - 5 |
| Weitere Parfümbestandteile | | <1 |

### Beispiel 4: Grundlagen der Bestimmung der olfaktorischen Wirkung

### 4.1 Methode

Die wissenschaftliche Evaluierung von aromatherapeutischen Wirkungen steht erst am Anfang der Entwicklung (Maria Lis-Balchin, Aromatherapy Science, Pharmaceutical Press, 2006).

Die am Lehrstuhl für Physiologische Psychologie der Universität Wuppertal innerhalb der Arbeitsgruppe von Professor Boucsein entwickelte Methodik des Objektiven Emotionalen Assessments (OEA) erlaubt eine Mehrebenen-Erfassung emotionaler Reaktionen von Probanden während der Beurteilung unterschiedlichster Produkte. Erfindungsgemäß wird die olfaktorische Wirkung auf die psychopsychologische Ausgangslage mit dem Objective Emotional Assessment (kurz: OEA) bestimmt.

Eisfeld, Wachter, Stürmer, Schaefer und Boucsein (SÖFW - Journal 4-2006, S. 84-90) beschreiben das Verfahren als "a unique methodological approach which is based on the measurement of psycho-physiological parameters like skin conductivity, heartbeat, blood circulation in the periphery and electrical muscle signals (...). All these physiological reactions are the result of unconscious mental processes in the brain (...). (...) from such measurements, the emotional state of the test persons can be inferred with high accuracy. (...) This approach was successfully introduced as »Objective Emotional Assessment« (OEA) in recent studies on cosmetic applications, and proved to be a suitable methodology for objective evaluation of emotional consumer response".

Beim OEA werden (1) der subjektive Eindruck (Gefühlskomponente) über Fragebogen erfasst, (2) physiologische Reaktionen (Aktivierungskomponente) mittels Parametern der elektrodermalen Aktivität (EDA) sowie Kennwerten aus dem Elektrokardiogramm (EKG) quantifiziert, und (3) die Verhaltensebene durch eine objektive Auswertung des mimischen Ausdrucks mit Hilfe von elektromyographischen (EMG) Messungen der Gesichtsmuskelaktivität beschrieben.

Eine mit der Methodik des Objective Emotional Assessment (OEA) durchgeführte Produktevaluation liefert als Ergebnis objektiv-quantifizierte Aussagen über die wahrgenommenen Produkteigenschaften sowie die durch die Interaktion mit dem Produkt beim Konsumenten ausgelöste emotionale Reaktion. Auf Grundlage von empirisch unter kontrollierten Laborbedingungen erhobenen Daten wird für jedes Produkt das durch dieses erzeugte spezifische psychophysiologische Emotionsprofil bestimmt.

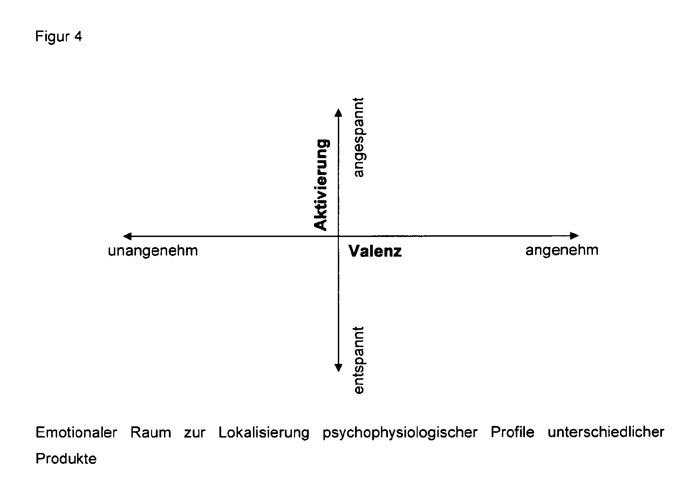

Die für die Untersuchung der Badeprodukte besonders relevanten Eigenschaften "Stimmungsaufhellung" und "Entspannung" lassen sich übersetzen in die beiden üblicherweise in der Psychologie zur Charakterisierung von emotionalen Reaktionen bzw. Zuständen verwendeten Dimensionen Valenz (angenehm - unangenehm) und Aktivierung (entspannt - angespannt). Die statistische Methode der Diskriminanzanalyse gestattet die Lage jedes getesteten Produktes auf diesen Dimensionen (d.h. die Ausprägung der durch das Produkt ausgelösten Empfindungen) aufgrund der spezifischen Ausprägungen der erfassten psychophysiologischen Emotionsindikatoren eindeutig zu lokalisieren und zufallskritisch abzusichern. Die Figur 4 veranschaulicht den emotionalen Raum, in dem die Lage der Produkte bestimmt wird. Paarweise Vergleiche der Produkte, bzw. die Bildung einer Rang-Reihenfolge aller Produkte bezogen auf die interessierenden Charakteristika sind ein zentraler Teil der Untersuchungsergebnisse.

Die besonderen Vorteile der verwendeten psychophysiologischen Methodik im Vergleich zu herkömmlichen (üblicherweise rein verbalen) Methoden der Produkttestung bestehen in der größeren Objektivität und geringeren Anfälligkeit für bewusste Verfälschung sowie in dem Potenzial auch automatisch ablaufende emotionale Reaktion valide abbilden zu können. Des weiteren wird durch die multivariante Erfassung unterschiedlicher Emotionskomponenten der holistische Wahrnehmungseindruck abgebildet. Im Vergleich zur selektiven Betrachtung einzelner, isolierter Produkteigenschaften berücksichtigt dieser Ansatz auch mögliche Interaktionen unterschiedlicher Eigenschaften, die in qualitativ andersartigen Wahrnehmungseindrücken resultieren können.

Eine genauere Beschreibung des OEA sowie die Ergebnisse von mit dieser Methodik durchgeführten experimentellen Untersuchungen finden sich in:
- Boucsein, W., Schaefer, F., Schwerdtfeger, A., Busch, P. & Eisfeld, W. (1999). Objective emotional assessment of foam. SÖFW-Journal, 125, 2-17.
- Boucsein, W. Schaefer, F., Kefel, M., Busch, P. & Eisfeld, W. (2002). Objective emotional assessment of tactile hair properties and their modulation by different product worlds. International Journal of Cosmetic Science, 24, 135-150.
- Boucsein, W., & Schaefer, F. (2007). Objective emotional assessment of industrial products. In J. Westerink, M. Ouwerkerk, T. Overbeek, F. Pasveer, & B. de Ruyter (Eds.), Probing Experience: From academic research to commercial propositions. Dordrecht: Springer.
- Eisfeld, W., Schaefer, F., Boucsein, W.& Stolz, C. (2005). Tracking Intersensory Properties of Cosmetic Products via Psycho-Physiological Assessment. IFSCC Magazine 8(1), 25-30.
- Eisfeld, W., Wachter, R., Stürmer, R., Schaefer, F., Boucsein, W. (2006). Perceivable Wellness Effects via a New Liposome Concept for Fabric Care. SÖFW-Journal, 132, 84-92.
- Eisfeld, W., Wachter, R., Schaefer, F., & Boucsein, W. (2007). Objective emotional assessment of perceivable wellness effects. Cosmetics & Toiletries, 122, 63-72.

### 4.2 Fragestellung

In einer ersten Untersuchung wurde grundsätzlich die positive olfaktorisch-vermittelte Wirkung der in den Beispielen 1-3 beschriebenen kosmetischen Reinigungs- und Pflegezusammensetzungen auf die Stimmung und den psycho-physiologischen Aktivierungsgrad nachgewiesenen. Als aktivierend/erfrischend hat sich hierbei insbesondere das Beispiel 3 herausgestellt. Bei diesem Beispiel wurde die als Parfümöl (PÖ) Zitronen-Melisse bezeichnete Mischung von ätherischen Ölen eingesetzt, die in der Zusammensetzung der Tabelle 7 beziehungsweise der Tabelle 2 entspricht.

Im Anschluss wurde eine zweite Untersuchung zur näheren Abklärung der Fragestellung konzipiert, ob diese Wirkung spezifisch für die verwendete spezielle Parfumöl-Kombination ist, oder auch beliebig durch (im weitesten Sinne alle üblichen) Parfumöle hervorgerufen wird. Hierzu wurde die Wirkung der kosmetischen Reinigungs- und Pflegezusammensetzung (PÖ) mit den Einzelwirkungen der drei wesentlichen darin enthaltenen ätherischen Ölen (Zitronenöl, Citronellaöl, Litsea Cubeba Öl) verglichen.

### 4.3 Durchführung

Die Einzelkomponenten Zitronenöl (Probe 1), Citronellaöl (Probe 2) und Litsea Cubeba Öl (Probe 3) wurden in jeweils drei verschiedenen Konzentrationen (niedrig/mittel/hoch), die kosmetische Reinigungs- und Pflegezusammensetzung mit der Parfümöl[PÖ]-Komponente Zitrone-Melisse, die die in Tabelle 2 bzw. Tabelle 7 näher aufgeführten Komponenten enthält (Probe 4) in der in vorangegangenen Studie verwendeten Konzentration dargeboten. Die olfaktorische Darbietung der vier Proben mit einer Dauer von jeweils 40 Sekunden erfolgte permutiert über die 16 weiblichen Probanden mit paralleler Registrierung der physiologischen Signale. Die subjektive Beurteilung der Proben erfolgte im direkten Anschluss an die Darbietung. Die physiologischen Reaktionen werden als Differenzwerte im Vergleich zur Ausgangslage (Baseline) dargestellt.

### 4.4 Ergebnisse

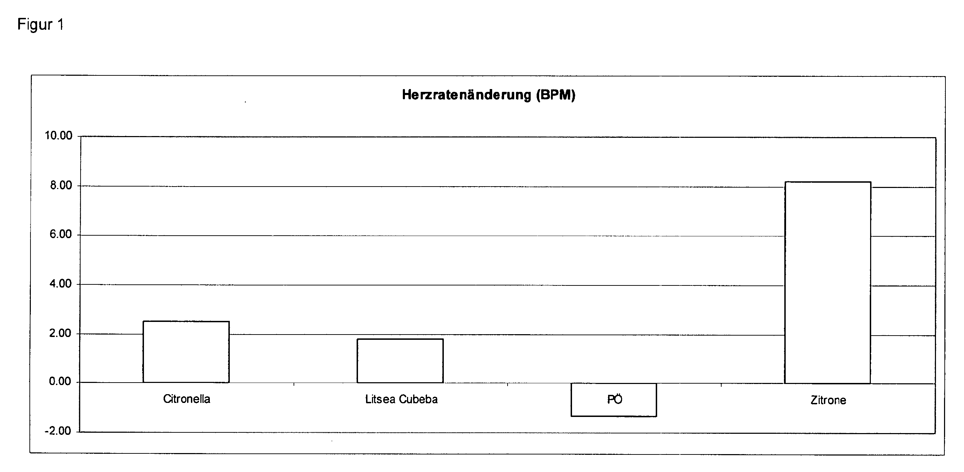

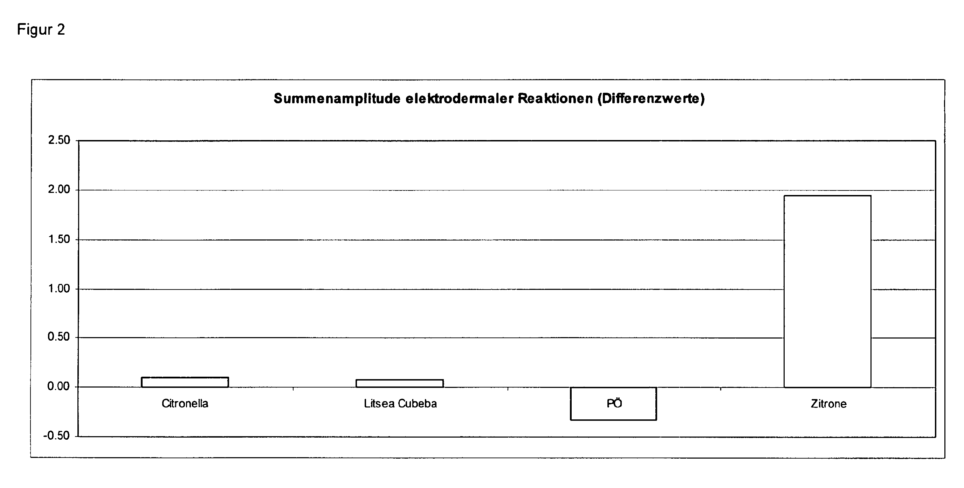

Summarisch betrachtet zeigt sich ein prinzipiell unterschiedliches peripher-physiologisches Reaktionsmuster für die kosmetische Reinigungs- und Pflegezusammensetzung (PÖ) im Vergleich zu den Einzelsubstanzen. Nur für erstere zeigt sich eine Verringerung der mittleren Herzrate und der Summenamplitude elektrodermaler Reaktionen (Figur 1 und 2).

Der Reaktionsverlauf der Herzrate reflektiert zum einen den allgemein beruhigenden Effekt der kosmetischen Reinigungs- und Pflegezusammensetzung. Des Weiteren steht dieser Verlauf, als phasische Reaktion auf einem Reiz in dem verwendeten Zeitintervall, auch für eine aktive Reizaufnahme und Hinwendung zum Reiz.

Die Verringerung der Summenamplitude elektrodermaler Reaktionen reflektiert einerseits zusätzlich den allgemein beruhigenden Effekt der kosmetischen Reinigungs- und Pflegezusammensetzung. Da ein starker Anstieg der Summenamplitude elektrodermaler Reaktionen besonders eine mit negativen Empfindungen einhergehende Erhöhung der emotionalen Aktivierung widerspiegelt, zeigt sich hier auch die positivere Valenz-Beurteilung (im Sinne von angenehm) der Zusammensetzung im Vergleich zu den Einzelsubstanzen.

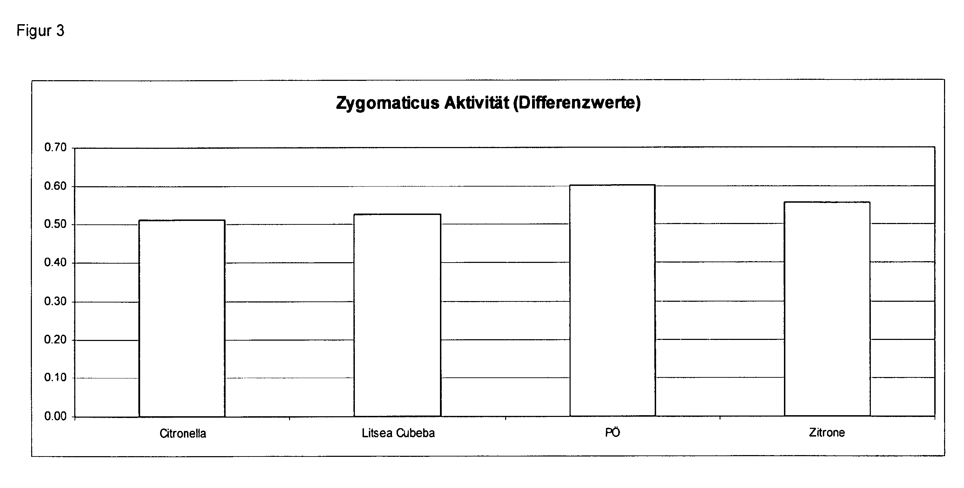

Konsistent mit diesen Reaktionsverläufen zeigt sich die positivere Beurteilung der kosmetischen Reinigungs- und Pflegezusammensetzung im Vergleich zu den Einzelsubstanzen, neben den Einschätzungen in Valenz-bezogenen Fragebogenitems (angenehm, ansprechend, ausgeglichen), in Entspannungs-bezogenen Items (beruhigend, entspannend), sowie in Aktivierungs-bezogenen Items (anregend, aktivierend, aufmerksamkeitserregend, belebend, erfrischend), auch in der Aktivität des Musculus zygomaticus major (Muskelgruppe, die beim Lächeln die Mundwinkel nach oben zieht). Diese Komponente des mimischen Ausdrucks, die bei positiven Empfindungen eine verstärkte Aktivität zeigt, war während der Darbietung der kosmetischen Reinigungs- und Pflegezusammensetzung mit der Parfümöl[PÖ]-Zusammensetzung am größten (Figur 3).

Die durch die erfindungsgemäße Zusammensetzung der kosmetischen Reinigungs- und Pflegezusammensetzung ausgelöste positive, olfaktorisch-vermittelte Wirkung auf die Stimmung und den psycho-physiologischen Aktivierungsgrad erwies sich somit im Rahmen der vorliegenden Untersuchung als spezifisch für die spezielle verwendete Parfumöl-Kombination und objektiv nachweisbar andersartig als die Einzelwirkungen der darin wesentlich enthaltenen ätherischen Öle.

### Beispiel 5 (Vergleichsbeispiel)

Wettbewerbsprodukt mit folgender Auslobung:
Gesundheitsbad
Wohltuende Entspannung vor dem Einschlafen.

Und setzt sich wie folgt zusammen:
INCI: Aqua, Sodium Laureth Sulfate, Polysorbate 20, Glycerin, PEG-6-Caprylic/Capric Glycerides, Parfum, Coco-Glucosides, Glyceryl Oleate, Valeriana officinalis, Lavandula angustifolia, Cymbopogon nardus, Humulus lupulus, Ethoxydiglycol, Propylene Glycol, Butylene Glycol, Lactic Acid, Glucose, Phenoxyethanol, Methylparaben, Butylparaben, Propylparaben, Isobutylparaben, Hexylcinnamal, Butyphenyl Methyprobional, Linalool, Limonene, Alpha-Isometyl lonone, Geraniol, Cl 42051

Um die Auswirkungen der Reinigungs- und Pflegezusammensetzung auf den Gemütszustand der Probanden objektiv messen zu können, wurden verschiedene Parameter bestimmt. Die Probanden erhielten zunächst eine Duftprobe der Zusammensetzung, dann wurden Veränderungen der Gesichtsmuskeln, Veränderungen der Herzschlagfrequenz, der peripheren Durchblutung und elektrodermalen Aktivität bestimmt.

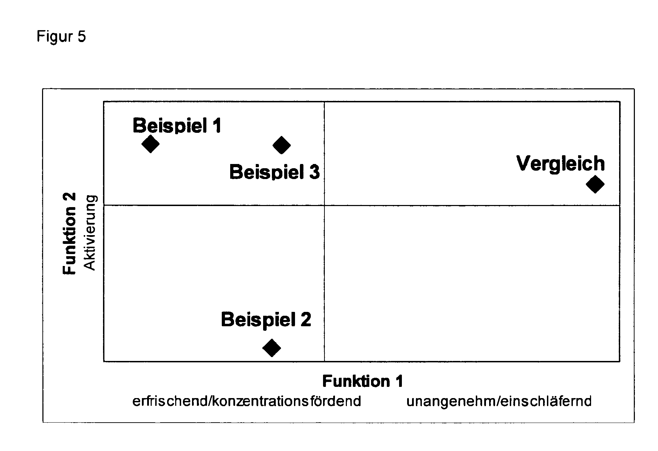

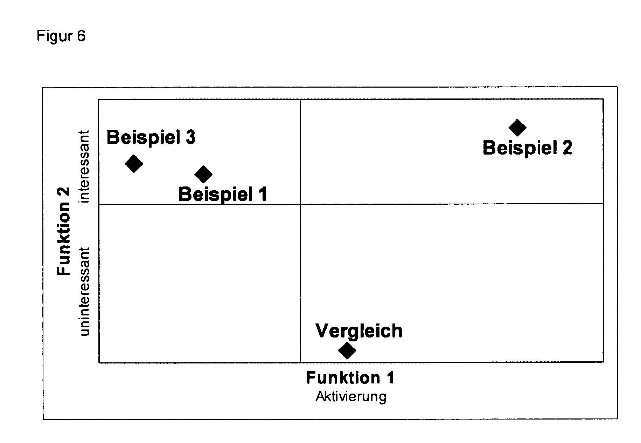

Mit Hilfe der im Abschnitt 4.1 näher dargelegten Methode wurden die Reinigungs- und Pflegezusammensetzung vorzugsweise Badezusätze gemäß Beispiel 1, 2 und 3 getestet und es wurde ein Vergleichstest mit einem Produkt gemäß Beispiel 5 durchgeführt. Dabei wurden zusammenfassend die in Figur 5 und 6 dargestellten Ergebnisse erhalten.

Figur 5 zeigt, dass die erfindungsgemäßen Beispiele alle eine erfrischende, konzentrationsfördernde Wirkung haben. Das Vergleichsbeispiel weist diese Wirkung dagegen nicht auf.

Figur 6 zeigt, dass sämtliche erfindungsgemäßen Zusammensetzungen einen interessanten Eindruck bei den Testpersonen hinterlassen, wohingegen das Vergleichsbeispiel einen uninteressanten und langweiligen Eindruck macht.

## Patentansprüche

1. Kosmetische Reinigungs- und/oder Pflegezusammensetzung mit messtechnisch nachgewiesener olfaktorischer Wirkung auf die psycho-physiologische Ausgangslage, die 30-70 Gew.-% mindestens eines Tensides und/oder eines Solubilisators, enthält, wobei die Zusammensetzung 0,1-20 Gew.-%, einer Mischung mehrerer ätherischer Öle und/oder einzelner natürlicher, naturidentischer oder synthetischer Duftstoffe mit olfaktorischer Wirkung enthält, **dadurch gekennzeichnet, dass** es sich bei diesen 0,1-20 Gew.-% der Mischung mehrerer ätherischer Öle um ein Gemisch aus von Citrusarten und Citronengras, das im Handel auch als Indische Melisse bezeichnet wird, gewonnenem ätherischem Öl, in Kombination mit Terpenen und Terpenoiden und Litsea Cubeba Öl handelt, wobei das ätherische Öl die nachfolgend aufgeführten Inhaltsstoffe in den angegebenen relativen Anteilen:
| Inhaltstoff | Anteil in Gew% |
|---|---|
| Terpenes and Terpenoids, limonen fraction | 10 - 50 |
| Linalyl acetat | 10 - 50 |
| Litsea Cubeba Fruit Oil | 10 - 50 |
| Bois de Rose Bresil Essence | 5 - 10 |
| Cornmint oil | 5 - 10 |
| Linalool | 5 - 10 |
| β-Pinen | 1 - 5 |
| α-Pinen | 0,1 - 1 |
| γ-Terpinene | 0,1 - 1 |
| Ocimene | 0,1 - 1 |
| Spearmint Oil | 0,1 - 1 |
| Terpinyl acetat | 0,1 - 1 |
oder in den angegebenen relativen Anteilen:
| Inhaltstoff | Anteil in Gew% |
|---|---|
| Terpenes and Terpenoids, limonen fraction | 10 - 50 |
| Citral | 10 - 50 |
| Litsea Cubeba Fruit Oil | 10 - 50 |
| Citronella Oil | 5 - 10 |
| Lemon Oil | 1 - 5 |
| Weitere Parfümbestandteile | <1 |
enthält und, dass sie 0,1-5 Gew.-% eines weiteren Pflanzenöls enthält.

2. Zusammensetzung nach Anspruch 1, **dadurch gekennzeichnet, dass** sie zwischen 35 und 55 Gew.-% eines Tensids oder einer Mischung verschiedener Tenside oder eines Solubilisators enthält.

3. Zusammensetzung nach einem der vorhergehenden Ansprüche, **dadurch gekennzeichnet, dass** sie 1-20 Gew.-% Glycerin enthält.

4. Zusammensetzung nach einem der vorhergehenden Ansprüche, **dadurch gekennzeichnet, dass** sie keine Konservierungsstoffe enthält.

5. Zusammensetzung nach einem der vorhergehenden Ansprüche, **dadurch gekennzeichnet, dass** sie keine Paraffine, Silikone und/oder Mineralöle enthält.

6. Zusammensetzung nach einem der vorhergehenden Ansprüche, **dadurch gekennzeichnet, dass** sie weiterhin einen oder mehrere Pflanzenextrakte enthält.

7. Zusammensetzung nach einem der vorhergehenden Ansprüche, **dadurch gekennzeichnet, dass** sie keine Bestandteile enthält, die von Tieren herstammen.

8. Zusammensetzung nach einem der vorhergehenden Ansprüche, **dadurch gekennzeichnet, dass** es kein Haarpflegemittel ist.

## Claims

1. A cosmetic cleaning and/or care composition having a metrologically proven olfactory effect on the psycho-physiological initial situation and containing 30-70% w/w of at least one tenside and/or solubilizer, wherein the composition contains 0.1-20% w/w of a blend of several essential oils and/or single native, nature-identical, or synthetic scents having an olfactory effect, **characterized in that** these 0.1-20% w/w of the blend of several essential oils are a mixture of essential oil obtained from types of citrus and lemon-grass, on the market also referred to as Indian melissa, in combination with terpenes and terpenoids and Litsea Cubeba oil, wherein the essential oil contains the ingredients listed below in the given relative proportions:
| Ingredient | proportion in % w/w |
|---|---|
| Terpenes and terpenoids, limonen fraction | 10 - 50 |
| Linalyl acetate | 10 - 50 |
| Litsea Cubeba Fruit Oil | 10 - 50 |
| Bois de Rose Bresil Essence | 5 - 10 |
| Cornmint oil | 5 - 10 |
| Linalool | 5 - 10 |
| β-Pinene | 1 - 5 |
| α-Pinene | 0.1 - 1 |
| γ-Terpinene | 0.1 - 1 |
| Ocimene | 0.1 - 1 |
| Spearmint Oil | 0.1 - 1 |
| Terpinyl acetate | 0.1 - 1 |
or in the given relative proportions:
| Ingredient | proportion in % w/w |
|---|---|
| Terpenes and terpenoids, limonen fraction | 10 - 50 |
| Citral | 10 - 50 |
| Litsea Cubeba Fruit Oil | 10 - 50 |
| Citronella Oil | 5 - 10 |
| Lemon Oil | 1 - 5 |
| further perfume components | <1 |
and that it contains 0.1-5% w/w of a further vegetable oil.

2. The composition according to claim 1, **characterized in that** it contains between 35 and 55% w/w of a tenside or a blend of different tensides or of a solubilizer.

3. The composition according to any one of the preceding claims, **characterized in that** it contains 1-20% w/w of glycerol.

4. The composition according to any one of the preceding claims, **characterized in that** it contains no preservatives.

5. The composition according to any one of the preceding claims, **characterized in that** it contains no paraffins, silicones, and/or mineral oils.

6. The composition according to any one of the preceding claims, **characterized in that** it further contains one or more plant extracts.

7. The composition according to any one of the preceding claims, **characterized in that** it contains no components originating from animals.

8. The composition according to any one of the preceding claims, **characterized in that** it is no hair-care product.

## Revendications

1. Composition de nettoyage et/ou de soin cosmétique avec effet olfactif prouvé selon la technique de mesure sur la situation de départ psycho-physiologique, qui contient 30 à 70 % en poids d'au moins un tensioactif et/ou un agent de solubilisation, dans laquelle la composition contient 0,1 à 20 % en poids d'un mélange de plusieurs huiles volatiles et/ou de différentes senteurs naturelles, identiques aux naturelles ou synthétiques avec effet olfactif, **caractérisée en ce que** ces 0,1 à 20 % en poids du mélange de plusieurs huiles volatiles sont un mélange d'huile volatile extraite de types de citrus et de la citronnelle, également désignée par mélisse indienne dans le commerce, en combinaison avec des terpènes et des terpénoïdes et de l'huile de Litsea Cubeba, dans laquelle l'huile volatile contient les composants cités ci-après dans les proportions relatives indiquées :
| Composant | Proportion en % en poids |
|---|---|
| Terpènes et terpénoïdes, fraction limonène | 10 - 50 |
| Acétate de linalyle | 10 - 50 |
| Huile du fruit de Litsea Cubeba | 10 - 50 |
| Essence de bois de rose du Brésil | 5 - 10 |
| Huile de menthe des champs | 5 - 10 |
| Linalol | 5 - 10 |
| β-pinène | 1 - 5 |
| α-pinène | 0,1 - 1 |
| y-terpinène | 0,1 - 1 |
| Ocimène | 0,1 - 1 |
| Huile de menthe en épi | 0,1 - 1 |
| Acétate de terpinyle | 0,1 - 1 |
ou dans les proportions relatives indiquées :
| Composant | Proportion en % en poids |
|---|---|
| Terpènes et terpénoïdes, fraction limonène | 10 - 50 |
| Citral | 10 - 50 |
| Huile du fruit de Litsea Cubeba | 10 - 50 |
| Huile de citronnelle | 5 - 10 |
| Huile de citron | 1 - 5 |
| Autres ingrédients de parfum | <1 |
et **en ce qu'**elle contient 0,1 à 5 % en poids d'une autre huile végétale.

2. Composition selon la revendication 1, **caractérisée en ce qu'**elle contient entre 35 et 55 % en poids d'un tensioactif ou d'un mélange de différents tensioactifs ou d'un agent de solubilisation.

3. Composition selon l'une quelconque des revendications précédentes, **caractérisée en ce qu'**elle contient 1 à 20 % en poids de glycérine.

4. Composition selon l'une quelconque des revendications précédentes, **caractérisée en ce qu'**elle ne contient aucun conservateur.

5. Composition selon l'une quelconque des revendications précédentes, **caractérisée en ce qu'**elle ne contient aucune paraffine, silicone et/ou huile minérale.

6. Composition selon l'une quelconque des revendications précédentes, **caractérisée en ce qu'**elle contient en outre un ou plusieurs extraits de plantes.

7. Composition selon l'une quelconque des revendications précédentes, **caractérisée en ce qu'**elle ne contient aucun composant d'origine animale.

8. Composition selon l'une quelconque des revendications précédentes, **caractérisée en ce qu'**elle n'est pas un produit de soin capillaire.
